# EUROPEAN PATENT SPECIFICATION

(11) **EP 3 224 390 B1**
(45) Date of publication and mention of the grant of the patent: **12.06.2019**
(21) Application number: 15804704.3
(22) Date of filing: 25.11.2015
(51) Int. Cl.: C25B 3/04, C07C 269/04

(54) **ELECTROCHEMICAL SYNTHESIS OF DICARBAMATES**
ELEKTROCHEMISCHE SYNTHESE VON DICARBAMATEN
SYNTHÈSE ÉLECTROCHIMIQUE DE DICARBAMATES

(30) Priority: 28.11.2014 IT RM20140694
(43) Date of publication of application: 04.10.2017
(73) Proprietor: Covestro Deutschland AG, 51373 Leverkusen (DE)
(72) Inventor: RICHTER, Frank, 51373 Leverkusen (DE); HECKROTH, Heike, 51519 Odenthal (DE); TRIEU, Vinh, 50679 Köln (DE); FEROCI, Marta, 00162 Rom (IT); FORTE, Gianpiero, 04023 Formia (IT); INESI, Achille, 00141 Rom (IT); CHIAROTTO, Isabella, 00137 Rom (IT)
(74) Representative: Levpat
(86) International application number: PCT/EP2015/077695
(87) International publication number: WO 2016/083475

(56) References cited:
- ULRICH HESS ET AL: "Reduktive elektrokatalytische Carboxylierung primärer und sekundärer aliphatischer Amine", ZEITSCHRIFT FÜR CHEMIE, vol. 28, no. 10, 1 October 1988 (1988-10-01), pages 372-373, XP055202789, DOI: 10.1002/zfch.19880281014 cited in the application
- DONG FANG NIU ET AL: "Nickel-catalyzed coupling of CO2 and amines: improved synthesis of carbamates", APPLIED ORGANOMETALLIC CHEMISTRY, vol. 21, no. 11, 1 November 2007 (2007-11-01), pages 941-944, XP055202857, ISSN: 0268-2605, DOI: 10.1002/aoc.1314 cited in the application

## Description

Several chemical routes for the production of O-alkyl carbamates as precursors for isocyanates are known, e.g. by reaction of primary amines with urea as the carbonyl source and an alcohol and evolution of ammonia. Employing an electrochemical route has the advantage that CO₂ as sustainable raw material can be used directly as the carbonyl source and no ammonia is produced as a side-product.

It is known that mono O-alkyl-carbamates can be synthesized electrochemically from mono amines with CO₂ as the carbonyl source and an alkyl halide as alkylating agent. The synthesis of bis-O-alkyl-carbamates from primary diamines is not described in the prior art.

Furthermore, the general disadvantage of the electrochemical routes of the state of the art is that ethyl chloride (Zeitschrift fuer Chemie 1988, 28, 372-373 and Pharmazie 1992, 47, 848-851) or ethyl iodide (Chem. Commun., 1996, 2575---2576; J. Org. Chem. 2007, 72, 200-203; J. Org. Chem. 1997, 62, 6754---6759; Electrochim. Acta 2011, 56, 5823---5827; Tetrahedron Lett. 2000, 41, 963---966; J. Org. Chem. 2003, 68, 1548---1551 *and* Appl. Organometal. Chem. 2007, 21, 941---944.), respectively, are used as alkylating agent. Both are impractical to use on a large scale for price, boiling point, and toxicological considerations.

The reactions employing ethyl chloride as alkylating agent have been performed in dimethyl formamide (DMF) containing 0.05 moles per liter of tetrabutylammonium iodide (TBAI) as the supporting electrolyte, on an Hg electrode. Both the solvent and the electrode material are especially impractical on an industrial scale (Zeitschrift fuer Chemie 1988, 28, 372-373 and Pharmazie 1992, 47, 848-851).

The reactions employing ethyl iodide as alkylating agent have been performed with a large excess of the alkylating agent, typically 3 to 5 fold over amine, which makes this processes particularly unattractive in view of the high price of the iodide.

Generally, the final thermal splitting of the O-alkyl-carbamates in the manufacture of isocyanates would furnish low boiling ethanol as a by-product which makes the whole process difficult to operate under industrial conditions.

Therefore, higher boiling alcohols like n-butanol would be more preferred. This leads to the prerequisite in the electrochemical process to employ higher boiling and thus longer chained alkylating agents, e.g. n-butyl chloride.

In preliminary experiments (see comparative example 1) it has been found, that n-butyl chloride is much less reactive compared to both ethyl chloride and ethyl iodide and the reaction is very sluggish providing for less than 10% of the target O-butyl-N-alkylcarbamate.

Therefore, the aim of the present invention was to develop a process that can also be conducted on an industrial scale with the use of higher alkylating agents.

Surprisingly, it has been found that butyl chloride can be activated at room temperature by addition of an iodide source, as e.g. tetrabutyl ammonium iodide (TBAI) or tetraethyl ammonium iodide (TEAI). That increases the isolated yield up to 21% (TBAI) or 40% (TEAI), respectively. Most surprisingly, it has been found that butyl chloride can be activated for this synthesis by heat treatment. In this case, the addition of an iodide source is not necessary. The posttreatment with heat yields up to >87% of the desired bis-carbamates. When an iodide source, e.g. TBAI or TEAI are used and the synthesis is carried out under heat treatment the highest yields are reached (up to 95%).

Accordingly the present invention provides an electrochemical process for preparing bis-O-alkyl-carbamates from primary diamines with CO₂ as carbonyl source, characterized in that at least one alkyl halide with at least 3 C-atoms in the alkyl group is used as alkylating agent in the presence of at least one iodide source and that the process is carried out at 10 to 215 °C, preferably 20 to 215°C, more preferably at 60 to 21 5°C, most preferably at 60 to 195°C, utmost particularly preferred at 60 to 150 °C.

Suitable iodide sources are e.g. symmetrically substituted tetrorganyl ammonium iodides, e.g. tetramethyl-, tetraethyl-, tetrapropyl-, tetrabutyl-, and tetraphenyl ammonium iodides, unsymmetrically substituted tetraorganyl ammonium iodides e.g. triphenyl methyl ammonium iodide, trimethyl benzyl ammonium iodide, triethyl methyl ammonium iodide, or ethyltrimethylammonium iodide. Preferred tetraorganyl ammonium iodides are tetrabutyl ammonium iodide (TBAI) and tetraethyl ammonium iodide (TEAI).

Further suitable iodide sources are e.g. sodium iodide or potassium iodide. When sodium iodide and/or potassium iodide are used, they are preferably used in pure form or in association with 15-crown-5 or 18-crown-6. Thereby, their solubility can be enhanced.

The iodide source is used in an amount of at least 0,5 mol-%, related to the alkyl halide/ the sum of the alkyl halides. If two or more iodide sources are used the above specified amount of at least 0,5 mol-% refers to the sum of these iodides.

Preferably the following amounts of iodide/es related to the alkyl halide/es are used (in rising preference): at least 0.5 mol-%, 0.5 to 80 mol-%, 0.5 to 50 mol-%, 0.5 to 20 mol-%, 0.5 to 10 mol-%, 0.5 to 5 mol-%, 1 to 5 mol-%.

In an alternative embodiment of the invention at least one alkyl halide with at least 3 C-atoms in the alkyl group is used and the alkyl halide is activated by heat treatment at 60 to 215°C, preferably at 60 to 195°C, more preferably at 60 to 150°C. In this case no iodide source is added.

Suitable primary diamines for the above described process are aliphatic and aromatic diamines, preferred primary diamines are 1,6-diaminohexane, 4,4'-methylenebis(cyclohexylamine), 5-Amino-1,3,3-trimethylcyclohexanemethylamine, 1,4-diaminobenzene, 2,4-diaminotoluene.

The alkyl halides, which are used in the inventive process, have alkyl groups with at least 3 C-atoms, preferably with at least 4 C-atoms.

Examples for suitable alkyl halides are alkyl iodides and alkyl chlorides such as, n- or iso-propyl iodide, n- or iso-propyl chloride, n-, iso- or t-butyl iodide and n-, iso- or t-butyl chloride; preferred alkyl halides are alkyl chlorides, particularly preferred is n-, iso- or t-butyl chloride, most preferred is n-butyl chloride.

According to the understanding of the present invention alkyl iodides do not belong to the iodide sources.

The alkyl halides can be used in different amounts. However, it is further preferred that the alkyl halides are used in such amounts that the groups of the alkyl halides are in a ≤ 2.5 molar excess, preferably in a ≤ 2.0 molar excess, over the amino groups of the primary diamines. Thereby, the efficiency of the whole process can be further improved.

The syntheses are carried out in solvents. Suitable solvents are for example DMF, DMSO, 1,2-dimethoxy ethane or N-methyl-2-pyrrolidone. Another suitable solvent is acetonitrile. Further suitable solvents are ionic liquids, for example 1-butyl-3-methylimidazolium tetrafluoroborate.

In the cases wherein the activation of the alkyl halide is carried out under heat treatment, it is preferred to work under reflux.

Moreover the syntheses can be carried out under normal pressure (1 atm), reduced pressure or increased pressure, preferably under normal pressure (1 atm) or increased pressure. The temperatures given above refer to syntheses which are carried out under normal pressure.

Suitable electrode materials are for example copper, platinum, zinc, nickel, iron, steel, graphite, glassy carbon and lead.

### Examples

### Details for the measurements of analytics:

Electrolyses under galvanostatic control were carried out with an Amel 552 potentiostat equipped with an Amel 721 integrator; ¹H and ¹³C NMR spectra were recorded on a Bruker AC 200 spectrometer using CDCl₃ as internal standard.

*Electrochemical cell.*

The cell is composed of a beaker, which contains the copper cathode (*A* ≃ 10 cm²), covered with a three-necked lid. A glass tube, equipped with a glass frit, is filled with methylcellulose gel and contains the platinum anode (apparent area: *A* ≃ 1 cm²). The gas inlet is provided by a pipette which dips into the solution, while the gas outlet is ensured by a side-necked plug.

*Methylcellulose gel.* Prepared with 1M solution of tetraethylammonium chloride in DMF (7g methylcellulose/100 mL solution).

### I. General protocol for the preparation of alkyl carbamate diesters (without addition of an iodide-source)

### Inventive: under heat treatment - comparative: no heat treatment.

In a two-compartment electrochemical cell typically 20 to 30 mL of 0.1 M of tetraethylammonium tetrafluoroborate in CH₃CN were added to the cathodic compartment and gaseous CO₂ was bubbled in. A 25 mA current was applied (*J* = 25 mA·cm⁻²) until the consumption of 300 C (3.0 Faradays per mole of amino-group). The current was stopped, the anodic compartment was removed and the solution was flushed with a nitrogen stream for 10 seconds. The diamine (0.5 mmol) was added to the catholyte and the solution was kept under nitrogen atmosphere while stirring. After 1 hour, the cathode was removed and n-butyl chloride (5.0 mmol) was added. In the control experiment (comparative example 1) the mixture was stirred overnight at room temperature. In the experiments according to the invention the reaction mixture was refluxed for 3 hours and subsequently stirred overnight at room temperature.

The catholyte was transferred thereafter into a round-bottom flask and the solvent was evaporated under reduced pressure. The remaining solid was extracted with ethyl acetate (3 times) and the organic layers were combined and evaporated in vacuo. The crude reaction was purified by flash column chromatography (silica gel, AcOEt:n-hexane) to afford the pure carbamate diesters.

### Dibutyl hexane-1,6-diyldicarbamate (Comparative example - inventive example)

White powder
Comparative example 1: 9% yield
Example 2, according to the invention: 82% yield
¹H-NMR (200 MHz, CDCl₃) *δ* 4.83 (bs, 2H), 4.00 (t, 4H), 3.12 (app. q, 4H), 1.58-1.26 (m, 16H, overlapped with H₂O signal), 0.89 (t, 6H); ¹³C-NMR (200 MHz, CDCl₃) *δ* 156.9, 64.5, 40.7, 31.1, 29.9, 26.3, 19.1, 13.7.
R*f* = 0.3 (n-hexane: ethyl acetate 7:3).

### Dibutyl (methylene-bis(cyclohexane-4,1,diyl))dicarbamate (Example 3, according to the invention)

Off-white waxy solid, ≥ 85% yield
¹H-NMR (200 MHz, CDCl₃) *δ* 4.80 (bd, 1H), 4.55 (bd, 1H), 4.02 (bt, 4H), 3.75 (bs, 1H), 3.39 (bs, 1H), 1.99-1.94 (m, 2H), 1.73-1.00 (m, 26H, overlapped with H₂O signal), 0.91 (t, 6H); ¹³C-NMR (200 MHz, CDCl3) *δ* 156.0, 64.5, 50.3, 46.9, 44.0, 42.9, 33.7, 33.6, 33.4, 32.7, 32.0, 31.1, 29.7, 28.0, 19.1, 13.8.
R*f* = 0.3 (*n*-hexane: ethyl acetate 8:2).

### Butyl((5-((butoxycarbonyl)amino)-1,3,3-trimethylcyclohexyl)methyl)carbamate (Example 4, according to the invention)

Yellowish oil, ≥ 87% yield
¹H-NMR (200 MHz, CDCl3) *δ* 4.77 (bt, 1H), 4.50 (bd, 1H), 4.07-4.00 (m, 4H), 3.77 (bs, 1H), 3.26 (d, *J* = 6.2 Hz, 0.4H), 2.9 (d, *J* = 6.6 Hz, 1.5H), 1.74-1.16 (m, 11H, overlapped with H₂O signal), 1.05 (app s, 6H), 0.95-0.88 (m, 12H);
¹³C-NMR (200 MHz, CDCl3) *δ* 157.2, 157.1*,156.0, 64.7, 64.5, 54.8, 47.5*, 47.1, 46.4, 44.5, 42.7*, 41.9, 36.4, 35.0, 31.9, 31.8*, 31.1, 29.7, 27.6, 23.2, 19.1, 17.7*, 13.8, 12.3*.
R*f* = 0.3 and 0.2 - pair of diastereomers - (n-hexane: ethyl acetate 8:2)
* *minor diastereomers*

### Dibutyl (1,3-phenylenebis(methylene))dicarbamate. (Example 5, according to the invention)

White solid, 82% yield
¹H NMR (200 MHz, CDCl3) *δ* 7.22-7.11 (m, 4H), 5.41 (bs, 2H), 4.24 (d, *J* = 5.8 Hz, 4H), 4.01 (t, *J* = 6.5 Hz, 4H), 1.58-1.47 (m, 4H), 137-1.25 (m, 4H), 0.88 (t, *J* = 7.2 Hz, 6H); ¹³C NMR (200 MHz, CDCl3) *δ* 156.9, 139.2, 128.8, 126.4, 64.8, 44.8, 31.0, 19.0, 13.7. *Rf* = 0.1 (*n-*hexane: ethyl acetate 8:2).

### Synthesis of dibutyl hexane-1,6-diyldicarbamate with butyl chloride in DMF

In a two-compartment electrochemical cell (copper cathode and platinum anode) 25 mL of 0.1 M of tetraethylammonium tetrafluoroborate in DMF were added to the cathodic compartment and gaseous CO₂ was bubbled in. A 25 mA current was applied (*J* = 25 mA·cm⁻²) until the consumption of 300 C (3.0 Faradays per mole of amino-group). The current was stopped, the anodic compartment and the copper cathode were removed and the solution was flushed with a nitrogen stream for 10 minutes. The diamine (0.5 mmol hexamethylenediamine) was added to the catholyte and the solution was kept under nitrogen atmosphere while stirring. After 1 hour, *n*-butyl chloride (5.0 mmol) was added and the reaction mixture was kept for 3 hours at 130°C and subsequently stirred overnight at room temperature. The catholyte was transferred thereafter into a round-bottom flask and the solvent was evaporated under reduced pressure. The remaining solid was extracted with ethyl acetate (3 times) and the organic layers were combined and evaporated in vacuo. The crude reaction was purified by flash column chromatography (silica gel, AcOEt:*n*-hexane) to afford the pure carbamate diester in 95 % yield.

### II. Preparation of alkyl carbamate diesters (under use of ammonium iodide)

### Synthesis of butyl dicarbamates with butyl chloride (TBAI, catalytic amount) - room temperature (Example 6 - inventive)

In a two-compartment electrochemical cell typically 20 to 30 mL of 0.1 M of tetraethylammonium tetrafluoroborate in CH₃CN were added to the cathodic compartment and gaseous CO₂ was bubbled in. A 25 mA current was applied (*J* = 25 mA·cm⁻²) until the consumption of 300 C (3.0 Faradays per mole of amino-group). The current was stopped, the anodic compartment was removed and the solution was flushed with a nitrogen stream for 10 seconds. Hexamethylenediamine (0.5 mmol) was added to the catholyte and the solution was kept under nitrogen atmosphere while stirring. After 1 hour, the cathode was removed and n-butyl chloride (5.0 mmol) was added along with tetrabutylammonium iodide (1-5 mol%). The solution was allowed to stand overnight at room temperature under constant stirring. The catholyte was transferred into a round-bottom flask and the solvent was evaporated under reduced pressure. The remaining solid was extracted with ethyl acetate (3 times) and the organic layers were combined and evaporated in vacuo. The crude reaction was purified by flash column chromatography (silica gel, hexane:AcOEt 8:2) to afford the pure carbamate diester as a white powder in 21% yield.

### Synthesis of butyl dicarbamates with butyl chloride (TBAI, catalytic amount) - 80 °C (Example 7 - inventive).

In a two-compartment electrochemical cell typically 20 to 30 mL of 0.1 M of tetraethylammonium tetrafluoroborate in CH₃CN were added to the cathodic compartment and gaseous CO₂ was bubbled in. A 25 mA current was applied (J = 25 mA·cm⁻²) until the consumption of 300 C (3.0 Faradays per mole of amino-group). The current was stopped, the anodic compartment was removed and the solution was flushed with a nitrogen stream for 10 seconds. Hexamethylenediamine (0.5 mmol) was added to the catholyte and the solution was kept under nitrogen atmosphere while stirring. After 1 hour, the cathode was removed and n-butyl chloride (5.0 mmol) was added along with tetrabutylammonium iodide (1-5 mol%). The solution was heated to 80 °C, then the catholyte was transferred into a round-bottom flask and the solvent was evaporated under reduced pressure. The remaining solid was extracted with ethyl acetate (3 times) and the organic layers were combined and evaporated in vacuo. The crude reaction was purified by flash column chromatography (silica gel, hexane:AcOEt 8:2) to afford the pure carbamate diester as a white powder in > 90% yield.

### Synthesis of butyl dicarbamates with butyl chloride (TEAI as supporting electrolyte) - room temperature (Example 8 - inventive).

In a two-compartment electrochemical cell typically 20 to 30 mL of 0.1 M of tetraethylammonium iodide in CH₃CN were added to the cathodic compartment and gaseous CO₂ was bubbled in. A 25 mA current was applied (*J* = 25 mA·cm⁻²) until the consumption of 300 C (3.0 Faradays per mole of amino-group). The current was stopped, the anodic compartment was removed and the solution was flushed with a nitrogen stream for 10 seconds. Hexamethylenediamine (0.5 mmol) was added to the catholyte and the solution was kept under nitrogen atmosphere while stirring. After 1 hour the cathode was removed, n-butyl chloride (5.0 mmol) was added and the solution was allowed to stand overnight at room temperature under constant stirring. The catholyte was transferred into a round-bottom flask and the solvent was evaporated under reduced pressure. The remaining solid was extracted with ethyl acetate (3 times) and the organic layers were combined and evaporated in vacuo. The crude reaction was purified by flash column chromatography (silica gel, hexane:AcOEt 8:2) to afford the pure carbamate diester as a white powder in about 40% yield.

### Synthesis of butyl dicarbamates with butyl chloride (TEAI as supporting electrolyte) - 80 °C (Example 9 - inventive).

In a two-compartment electrochemical cell typically 20 to 30 mL of 0.1 M of tetraethylammonium iodide in CH₃CN were added to the cathodic compartment and gaseous CO₂ was bubbled in. A 25 mA current was applied (*J* = 25 mA·cm⁻²) until the consumption of 300 C (3.0 Faradays per mole of amino-group). The current was stopped, the anodic compartment was removed and the solution was flushed with a nitrogen stream for 10'. Hexamethylenediamine (0.5 mmol) was added to the catholyte and the solution was kept under nitrogen atmosphere while stirring. After 1 hour the cathode was removed, n-butyl chloride (5.0 mmol) was added and the solution was heated at 80 °C for 2 hours. The catholyte was transferred into a round-bottom flask and the solvent was evaporated under reduced pressure. The remaining solid was extracted with ethyl acetate (3 times) and the organic layers were combined and evaporated in vacuo. The crude reaction was purified by flash column chromatography (silica gel, hexane:AcOEt 8:2) to afford the pure carbamate diester as a white powder in > 95 % yield.

**Dibutyl hexane-1,6-diyldicarbamate.**¹H NMR (200 MHz, CDCl3) *δ* 4.83 (bs, 2H), 4.00 (t, 4H), 3.12 (app. q, 4H), 1.58-1.26 (m, 16H, overlapped with H2O signal), 0.89 (t, 6H); ¹³C NMR (200 MHz, CDCl3) *δ* 156.9, 64.5, 40.7, 31.1, 29.9, 26.3, 19.1, 13.7. R*f* = 0.3 (n-hexane: ethyl acetate 7:3).

### Synthesis of dibutyl hexane-1,6-diyldicarbamate with butyl chloride (TBAI, catalytic amount) in DMF

In a two-compartment electrochemical cell (copper cathode and platinum anode) 25 mL of 0.1 M of tetraethylammonium tetrafluoroborate in DMF were added to the cathodic compartment and gaseous CO₂ was bubbled in. A 25 mA current was applied (*J* = 25 mA·cm⁻²) until the consumption of 300 C (3.0 Faradays per mole of amino-group). The current was stopped, the anodic compartment and the copper cathode were removed and the solution was flushed with a nitrogen stream for 10 minutes. The diamine (0.5 mmol hexamethylenediamine) was added to the catholyte and the solution was kept under nitrogen atmosphere while stirring. After 1 hour, *n*- butyl chloride (5.0 mmol) was added along with tetrabutylammonium iodide (3 mol %) and the reaction mixture was kept for 3 hours at 130°C and subsequently stirred overnight at room temperature. The catholyte was transferred thereafter into a round-bottom flask and the solvent was evaporated under reduced pressure. The remaining solid was extracted with ethyl acetate (3 times) and the organic layers were combined and evaporated in vacuo. The crude reaction was purified by flash column chromatography (silica gel, AcOEt:*n*-hexane) to afford the pure carbamate diester in 93 % yield.

### III. Example for synthesis in cell without the gel

### Pseudo-undivided cell configuration.

**Electrochemical cell.** The cell is an H-type glass tube endowed with a glass frit (porosity: 2). Each of the two sides (internal diameter = 1.4 cm) contains one electrode: a copper bar - as the cathode- and a glassy carbon bar -as the anode- (A = 3 cm², depending on the amount of solvent).
The gas inlet is provided by glass pipettes on both sides of the cell.

### General protocol to alkyl carbamate diesters in a pseudo-undivided cell

In an H-type electrochemical cell (as described above) typically 10 mL of 0.1 M of tetraethylammonium chloride in CH₃CN were added to both the sides. Gaseous CO₂ was bubbled to the cathodic side, while nitrogen to the anodic one. A 50 mA current was applied *J* = 15 mA·cm⁻² until the consumption of 300 C (3.0 Faradays per mole of amino-group).* The current was stopped, the electrodes removed and the solution in the anodic side was replaced with fresh 0.1 M of tetraethylammonium chloride in CH₃CN. The catholyte was flushed with a nitrogen stream for 10' before adding the diamine (0.5 mmol) and then the solution was kept under nitrogen atmosphere while stirring. After 1 hour, the alkylating agent (5.0 mmol) was added. When butyl iodide was used, the solution was allowed to stand overnight at room temperature under constant stirring while, in the case of n-butyl chloride, the reaction mixture was refluxed for 3 hours and subsequently stirred overnight at room temperature. The catholyte was transferred into a round-bottom flask and the solvent was evaporated under reduced pressure. The remaining solid was extracted with ethyl acetate (3 times) and the organic layers were combined and evaporated in vacuo. The crude reaction was purified by flash column chromatography (silica gel, AcOEt:n-hexane) to afford the pure carbamate diesters.
* during the electrolysis, supplement of tetraethylammonium chloride in the anodic side was required to avoid solvent migration towards the cathode.

## Claims

1. Electrochemical process for preparing bis-O-alkyl-carbamates from primary diamines with CO₂ as carbonyl source, **characterized in that** at least one alkyl halide with at least 3 C-atoms in the alkyl group is used as alkylating agent in the presence of at least one iodide source and that the process is carried out at 10 to 215 °C, wherein the iodide source is not an alkyl iodide.

2. Electrochemical process according to claim 1, wherein the process is carried out at 20 to 215°C, preferably at 60 to 215°C, more preferably a t 60 to 195°C and most preferably at 60 to 150°C.

3. Electrochemical process according to claim 1 or 2, wherein the iodide source/es is/are used in an amount of at least 0,5 mol-%, more preferably of 0.5 to 5 mol-%, most preferably of 1 to 5 mol-% related to the alkyl halide/ the sum of the alkyl halides.

4. Electrochemical process according to any of claims 1 to 3, wherein the iodide source/es is/are symmetrically-substituted tetralkyl ammonium iodides, asymmetrically-substituted tetralkyl ammonium iodides and/or sodium iodide, preferably in pure form or in association with 15-crown-5 or 18-crown-6, and/or potassium iodide, preferably in pure form or in association with 15-crown-5 or 18-crown-6.

5. Electrochemical process according to any of claims 1 to 3, wherein the iodide source(s) is/are tetrabutyl ammonium iodide (TBAI) and/or tetraethyl ammonium iodide (TEAI).

6. Electrochemical process for preparing bis-O-alkyl-carbamates from primary diamines with CO₂ as carbonyl source, **characterized in that** at least one alkyl halide with at least 3 C-atoms in the alkyl group is used and the alkyl halide is activated by heat treatment at 60 to 215°C, preferably at 60 to 195°C, more prefer ably at 60 to 150°C.

7. Process according to any of claims 1 to 6, wherein 1,6-diaminohexane, 4,4'-methylenebis(cyclohexylamine), 5-Amino-1,3,3-trimethylcyclohexanemethyl-amine, 1,4-diaminobenzene and/or 2,4-diaminotoluene are used as primary diamine/s.

8. Process according to any of claims 1 to 7, wherein alkyl iodides and/or alkyl chlorides, preferably alkyl chlorides, are used as alkyl halides.

9. Process according to any of claims 1 to 7, wherein n-, iso- and/or t-butyl chloride, preferably n-butyl chloride, is used as alkyl halide.

10. Process according to any of claims 1 to 9, wherein the synthesis is carried out in a solvent.

11. Process according to claim 10, wherein DMF, DMSO, 1,2-dimethoxy ethane or N-methyl-2-pyrrolidone is used as solvent.

12. Process according to claim 10, wherein acetonitrile is used as solvent.

13. Process according to any of claims 10 to 12, wherein the heat treatment is carried under reflux.

14. Process according to any of claims 10 to 13, wherein the synthesis is carried out under normal pressure (1 atm) or increased pressure.

## Patentansprüche

1. Elektrochemisches Verfahren zur Herstellung von Bis-O-alkylcarbamaten aus primären Diaminen mit CO₂ als Carbonylquelle, **dadurch gekennzeichnet, dass** mindestens ein Alkylhalogenid mit mindestens drei C-Atomen in der Alkylgruppe als Alkylierungsmittel in Gegenwart von mindestens einer Iodquelle verwendet wird und dass das Verfahren bei 10 bis 215°C durchgeführt wird, wobei es sich bei der Iodquelle nicht um ein Alkyliodid handelt.

2. Elektrochemisches Verfahren nach Anspruch 1, wobei das Verfahren bei 20 bis 215°C, vorzugsweise bei 60 bis 215°C, weiter bevorzugt bei 60 bis 195°C und ganz besonders bevorzugt bei 60 bis 150°C durchgeführt wird.

3. Elektrochemisches Verfahren nach Anspruch 1 oder 2, wobei die Iodquelle/die Iodquellen in einer Menge von mindestens 0,5 Mol-%, weiter bevorzugt 0,5 bis 5 Mol-%, ganz besonders bevorzugt 1 bis 5 Mol-%, bezogen auf das Alkylhalogenid/die Summe der Alkylhalogenide, verwendet wird/werden.

4. Elektrochemisches Verfahren nach einem der Ansprüche 1 bis 3, wobei es sich bei der Iodquelle/den Iodquellen um symmetrisch substituierte Tetraalkylammoniumiodide, unsymmetrisch substituierte Tetraalkylammoniumiodide und/oder Natriumiodid, vorzugsweise in reiner Form oder in Assoziation mit 15-Krone-5 oder 18-Krone-6, und/oder Kaliumiodid, vorzugsweise in reiner Form oder in Assoziation mit 15-Krone-5 oder 18-Krone-6, handelt.

5. Elektrochemisches Verfahren nach einem der Ansprüche 1 bis 3, wobei es sich bei der Iodquelle/den Iodquellen um Tetrabutylammoniumiodid (TBAI) und/oder Tetraethylammoniumiodid (TEAI) handelt.

6. Elektrochemisches Verfahren zur Herstellung von Bis-O-alkylcarbamaten aus primären Diaminen mit CO₂ als Carbonylquelle, **dadurch gekennzeichnet, dass** mindestens ein Alkylhalogenid mit mindestens drei C-Atomen in der Alkylgruppe verwendet wird und das Alkylhalogenid durch Wärmebehandlung bei 60 bis 215 °C, vorzugsweise bei 60 bis 195 °C, weiter bevorzugt bei 60 bis 150 °C, aktiviert wird.

7. Verfahren nach einem der Ansprüche 1 bis 6, bei dem als primäres Diamin/primäre Diamine 1,6-Diaminohexan, 4,4'-Methylenbis(cyclohexylamin), 5-Amino-1,3,3-trimethylcyclohexanmethylamin, 1,4-Diaminobenzol und/oder 2,4-Diaminotoluol verwendet wird/werden.

8. Verfahren nach einem der Ansprüche 1 bis 7, wobei als Alkylhalogenide Alkyliodide und/oder Alkylchloride, vorzugsweise Alkylchloride, verwendet werden.

9. Verfahren nach einem der Ansprüche 1 bis 7, wobei als Alkylhalogenid n-, iso- und/oder t-Butylchlorid, vorzugsweise n-Butylchlorid, verwendet wird.

10. Verfahren nach einem der Ansprüche 1 bis 9, wobei die Synthese in einem Lösungsmittel durchgeführt wird.

11. Verfahren nach Anspruch 10, wobei als Lösungsmittel DMF, DMSO, 1,2-Dimethoxyethan oder N-Methyl-2-pyrrolidon verwendet wird.

12. Verfahren nach Anspruch 10, wobei als Lösungsmittel Acetonitril verwendet wird.

13. Verfahren nach einem der Ansprüche 10 bis 12, wobei die Wärmebehandlung unter Rückfluss durchgeführt wird.

14. Verfahren nach einem der Ansprüche 10 bis 13, wobei die Synthese unter Normaldruck (1 atm) oder erhöhtem Druck durchgeführt wird.

## Revendications

1. Procédé électrochimique pour la préparation de bis(carbamates de *O*-alkyle) à partir de diamines primaires avec du CO₂ en tant que source de carbonyle, **caractérisé en ce qu'**au moins un halogénure d'alkyle ayant au moins 3 atomes de C dans le groupe alkyle est utilisé en tant qu'agent alkylant en présence d'au moins une source d'ions iodure et **en ce que** le procédé est effectué à 10 à 215 °C, la source d'ions iodure n'étant pas un iodure d'alkyle.

2. Procédé électrochimique selon la revendication 1, le procédé étant effectué à 20 à 215 °C, de préférence à 60 à 215 °C, plus préférablement à 60 à 195 °C et le plus préférablement à 60 à 150 °C.

3. Procédé électrochimique selon la revendication 1 ou 2, dans lequel la ou les sources d'ions iodure sont utilisées en une quantité d'au moins 0,5 mol%, plus préférablement de 0,5 à 5 mol%, le plus préférablement de 1 à 5 mol% par rapport à l'halogénure d'alkyle/la somme des halogénures d'alkyle.

4. Procédé électrochimique selon l'une quelconque des revendications 1 à 3, dans lequel la ou les sources d'ions iodure sont des iodures de tétraalkylammonium substitués de façon symétrique, des iodures de tétraalkylammonium substitués de façon asymétrique et/ou l'iodure de sodium, de préférence sous forme pure ou en association avec le [15]-couronne-5 ou le [18]-couronne-6, et/ou l'iodure de potassium, de préférence sous forme pure ou en association avec le [15]-couronne-5 ou le [18]-couronne-6.

5. Procédé électrochimique selon l'une quelconque des revendications 1 à 3, dans lequel la ou les sources d'ions iodure sont l'iodure de tétrabutylammonium (TBAI) et/ou l'iodure de tétraéthylammonium (TEAI).

6. Procédé électrochimique pour la préparation de bis(carbamates de *O*-alkyle) à partir de diamines primaires avec du CO₂ en tant que source de carbonyle, **caractérisé en ce qu'**au moins un halogénure d'alkyle ayant au moins 3 atomes de C dans le groupe alkyle est utilisé et l'halogénure d'alkyle est activé par traitement thermique à 60 à 215 °C, de préférence à 60 à 195 °C, plus préférablement à 60 à 150 °C.

7. Procédé selon l'une quelconque des revendications 1 à 6, dans lequel du 1,6-diaminohexane, de la 4,4'-méthylènebis(cyclohexylamine), de la 5-amino-1,3,3-triméthylcyclohexaneméthylamine, du 1,4-diaminobenzène et/ou du 2,4-diaminotoluène sont utilisés en tant que la ou les diamines primaires.

8. Procédé selon l'une quelconque des revendications 1 à 7, dans lequel des iodures d'alkyle et/ou des chlorures d'alkyle, de préférence des chlorures d'alkyle, sont utilisés en tant qu'halogénures d'alkyle.

9. Procédé selon l'une quelconque des revendications 1 à 7, dans lequel du chlorure de *n*-butyle, du chlorure d'isobutyle et/ou du chlorure de *t*-butyle, de préférence du chlorure de *n*-butyle, sont utilisés en tant qu'halogénure d'alkyle.

10. Procédé selon l'une quelconque des revendications 1 à 9, dans lequel la synthèse est effectuée dans un solvant.

11. Procédé selon la revendication 10, dans lequel du DMF, du DMSO, du 1,2-diméthoxyéthane ou de la *N*-méthyl-2-pyrrolidone sont utilisés en tant que solvant.

12. Procédé selon la revendication 10, dans lequel de l'acétonitrile est utilisé en tant que solvant.

13. Procédé selon l'une quelconque des revendications 10 à 12, dans lequel le traitement thermique est effectué sous reflux.

14. Procédé selon l'une quelconque des revendications 10 à 13, dans lequel la synthèse est effectuée sous pression normale (1 atm) ou pression accrue.
